# EUROPEAN PATENT SPECIFICATION

(11) **EP 3 046 441 B1**
(45) Date of publication and mention of the grant of the patent: **08.08.2018**
(21) Application number: 14784192.8
(22) Date of filing: 15.09.2014
(51) Int. Cl.: A45D 19/00, A45D 19/02, A45D 34/04, A45D 40/26, A45D 40/28, A61F 7/00

(54) **PELTIER EFFECT DEVICE FOR CREATING A COOLING OR HEATING FEELING**
VORRICHTUNG MIT PELTIER-EFFEKT FÜR DIE SCHAFFUNG EINES KÜHL- ODER WÄRMEGEFÜHLS
DISPOSITIF AVEC D'EFFET PELTIER POUR CRÉER UNE SENSATION DE FRAÎCHEUR OU DE CHALEUR

(30) Priority: 18.09.2013 FR 1358953
(43) Date of publication of application: 27.07.2016
(73) Proprietor: L'OREAL, 75008 Paris (FR)
(72) Inventor: VIC, Gabin, 93400 Saint OUEN (FR); NUZZO, Stefania, F-75014 Paris (FR); WOODLAND, Frédéric, F-93220 Gagny (FR); LEGENDRE, Jean-Yves, F-75013 Paris (FR); ALLANIC, Thibault, 93400 Saint Ouen (FR)
(74) Representative: Bourdeau, Françoise
(86) International application number: PCT/EP2014/069617
(87) International publication number: WO 2015/039990

(56) References cited:
- FR-A1- 2 946 845
- FR-A1- 2 954 936
- US-A1- 2008 300 529

## Description

The present invention relates to devices having an application member that is able to create a cooling or heating feeling on keratin materials.

The expression "cosmetic product" is understood to mean any composition as defined in Council Directive 93/35/EEC of 14 June 1993.

In order to create a cooling feeling, the application FR 2 894 460 proposes using an application member which is cooled by being placed beforehand in a refrigerator or freezer or by being exposed to expansion of a compressed or liquefied gas or to an endothermic reaction.

The patent US 3 750 255 teaches using the thermal effusivity of an applicator ball in order to create a cooling feeling on application. An example is given with a solid steel ball having a diameter of 32 mm and a weight of 141 grams. The drawback of such a device is its weight, since in addition to the weight of the ball there is that of the container containing the product to be applied. The cost of the device may also be relatively high on account of the quantity of metal employed.

The patent application FR 2 915 972 relates to a dispenser having an end piece for storing heat, said end piece being made of a metal material such as Zamac®, the mass of which is between about 0.3 and 0.6 gram. Such a weight can prove to be too low for getting a sufficient cooling feeling.

The patent application FR 2 954 936 relates to a dispenser having an end piece for storing heat, said end piece being made of a metal material such as Zamac®, the mass of which is greater than 4 grams. Such a weight can also prove to be too low for getting a lasting cooling feeling.

The patent application WO2008/151260 discloses a Peltier effect device that is able to create a cooling or heating feeling on the skin or on an area of the body. Said document does not provide for the use of the device for treating the hair. It also does not provide for the use of the device for treating narrow areas of the body with precision.

The patent application FR 2 946 845 discloses a device for applying cosmetic composition to skin or hair, the device having a hand piece and a Peltier effect device. There is a need to further improve the prior art devices so as to have a device:
- that has a suitable size for treating a narrow and precise area of the body, including a lock of hair,
- that is capable of producing a cooling or heating feeling which is not too short-lived,
- that has an acceptable weight and cost, and
- is able to rapidly output the cold or heat without generating an internal short circuit.

Furthermore, there is a need for miniaturized appliances that are able to provide a cooling or heating feeling while producing a mechanical effect such as a massage.

The invention aims to meet this need and achieves this by virtue of a device for the cosmetic treatment of human keratin materials, according to claim 1. Preferably, the handpiece is designed to generate a thermal amplitude of less than or equal to 100°C between the keratin materials and the application surface for a duration of at least 5 minutes, in particular a thermal amplitude of less than or equal to 60°C for a duration of at least 5 minutes.

The device according to the invention is a miniaturized device that is capable of generating a cooling or heating feeling and has a precise application surface. The latter is specifically designed for the cosmetic treatment of limited areas such as the scalp, the roots of the hair and the periocular area. This device is also easy to handle. It is also easy to transport. The device according to the invention has at least one Peltier module.

The Peltier effect (also known as the *thermoelectric effect*) is a physical phenomenon relating to the movement of heat in the presence of an electric current. The Peltier effect is known per se. Conductors having different natures that are connected by junctions (contacts) are produced in the materials. One of the junctions cools while the other heats up. An electric current *I* is applied to the circuit, by placing for example a source of electric current between the materials, this causing heat *Q* to be released at one junction and heat to be absorbed at the other junction.

The name *"Peltier module"* is given to the assembly formed by the conductive materials and the junctions connecting them.

According to the invention, the *"surface of the Peltier module"* is understood to be the surface of the Peltier module that is located facing the application surface.

More advantageously, the ratio between the application surface of the device and the surface of the Peltier module is strictly greater than 1, preferably between 1.02 and 2.

By choosing this surface ratio, the excess energy generated by the Peltier effect can be emitted rapidly out of the device. Specifically, the excess energy generated by the Peltier effect is dissipated at the surface of the application member.

Thus, any overheating or excessive cooling is avoided. The thermal effect can last for longer. Internal short circuits are avoided. The consumer perceives a uniform and pleasant feeling of heat or cold. This feeling is more or less uniform over all of the keratin materials in contact with the application surface.

Advantageously, the device according to the invention has at least one radiator. Thus, the dissipation of the heat is optimized.

According to the invention, the Peltier effect is ensured between an internal mass and the application member. The weight ratio between the internal mass and the application member is between 1 and 15, preferably between 3 and 12, and more preferably between 5 and 10.

The advantages associated with the choice of such a mass ratio are:
- a sufficiently long feeling of heat or cold,
- a sensation that is perceived to be uniform, without peaks of cold or heat on the surface treated, and
- an application member temperature that is in accordance with the user's expectations.

By choosing an application member that is lighter than the internal mass, the cold and heat migrate better to the application surface. They are also generated with a sufficient reserve.

In accordance with the invention, the mass of the application member is advantageously between 2 grams and 5 grams, preferably between 2.1 grams and 4.5 grams, and more preferably between 2.2 grams and 4 grams.

More advantageously, the mass of the internal mass is advantageously between 12 grams and 25 grams, preferably between 15 grams and 22 grams, and more preferably between 16 grams and 19 grams. The emission of heat is optimized in these ranges of weights.

According to the invention, provision is made of a device that is capable of producing a sufficient cooling or heating feeling associated with the very structure of the device.

The expression "*application surface*" is understood to mean the surface that comes into contact with the keratin materials during application and which can carry a product. The application surface may for example be a front face of the application member when the device is viewed along the longitudinal axis.

The *"thermal amplitude"* represents a difference in temperatures in terms of absolute value. It is denoted δT. This difference in temperature corresponds to the difference, in terms of absolute value, between the temperature of the keratin materials treated and the application surface.

In order to determine the temperature of the keratin materials before and after they are brought into contact with the application surface of the device, use can be made of a thermal probe or an infrared camera.

Advantageously, the handpiece has a module for stabilizing the temperature of the application surface for at least 20 minutes. Thus, the user can benefit from a sufficient duration of the cooling or heating feeling.

More advantageously, the handpiece has a module for stabilizing the temperature of the application surface for at least 60 minutes.

### APPLICATION MEMBER

The application surface generally comprises an inorganic material. The latter may be chosen from metals, ceramics and glass, in particular metalized ceramics or glass.

The application surface may be produced at least partially, or even entirely, from a metal material.

The metal may be chosen from steel, stainless steel, aluminum, copper, silver, iron and alloys. The application member may be plated with another metal.

The material may be predominantly in a non-oxidized form.

The application surface may be completely inert from a chemical point of view with respect to the products and keratin materials.

The application surface may be covered with a varnish.

The application surface may be polished.

The application member may have a biocidal material, for example silver or copper, on its surface. Such a metal may be deposited in the form of a thin layer.

According to a first embodiment of the invention, the application surface is smooth. In this case, the device is particularly suitable for attenuating surface defects of an area of the body. It can in particular serve to temporarily attenuate lines or wrinkles on the body. Moreover, the distribution of heat or cold is homogeneous at every point of the surface of the body treated. This homogeneity is particularly appreciable if the device is used with a cosmetic product.

According to a second embodiment of the invention, the application surface has at least one raised element. In this case, the device may also serve to massage a chosen area of the body.

The raised element may in particular be chosen from spikes, balls and rollers.

The raised elements may themselves be smooth or have complementary raised areas.

Alternatively, the raised elements may be removable. It is thus possible to change the raised elements of the device, for example in order to modify their dimensions, their surface properties, or else their roughness.

The raised elements may comprise a thermoplastic material of the acrylic type, cellulose type, polycarbonate type, polyamide type, styrene type, polyolefin type, vinyl type or polyethylene terephthalate type and mixtures of said materials in a variable proportion, which are expanded or not expanded. The raised elements may also comprise one or more thermoplastic resins or one or more metals.

Advantageously, the device has a single Peltier module. All of the raised elements are in contact with the same Peltier module.

Thus, the device is less expensive and the emission of heat is controlled better.

The application member may be snap-fastened onto the handpiece. The application member may have an annular groove formed by machining, said groove being snap-fitted onto an annular bulge or an annular lip of the handpiece.

As a variant, the application member may be adhesively bonded to or snugly fitted onto the handpiece, or be fixed in some other way.

### HANDPIECE

Advantageously, the handpiece has a mass of less than 100 grams, preferably less than 80 grams. In this case, it is easy for any person to handle.

According to a first embodiment of the device, the handpiece is designed to generate heat at the application surface.

According to a second embodiment of the device, the handpiece is designed to generate cold at the application surface.

According to a third embodiment of the device, the handpiece is designed to generate heat or cold at the application surface, depending on the user's wishes. In general, the user can select heat or cold by means of a control button provided for this purpose.

The device according to the invention makes it possible to obtain a satisfactory cooling or heating feeling for the user. Cooling can have a decongestant and relaxing effect, being advantageously used during the application of a cosmetic product in order to improve the application of the product, in particular the comfort of application and the treating or makeup effect of the product. Heating can provide a feeling of well-being. It may also promote the penetration of some cosmetic active agents.

Advantageously, the handpiece is designed to be held between three fingers of one hand. The device is ergonomic. The user can use it for regions that are difficult to access. For example, the device can have the form of a pen.

More advantageously, the handpiece is configured such that the application surface reaches a temperature defined by the user in less than one minute. The device is thus rapidly operational.

Even more advantageously, the handpiece has an elongate shape, in particular a cylindrical shape. This geometry is especially suitable for the face, the scalp and the hair.

A further subject of the invention, according to another of its aspects, independently or in combination with the foregoing, is a cosmetic process comprising the employment of a device as described above.

Preferably, in the process according to the invention:
- the device is gripped between three fingers of one hand,
- the application surface is brought into contact with the keratin materials to be treated, and
- a thermal amplitude of greater than or equal to 5°C is generated between the keratin materials and the application surface for a duration of at least 5 minutes, in particular a thermal amplitude of greater than or equal to 20°C for a duration of at least 5 minutes.

The process according to the invention can be employed for any surface of the body such as the skin of the face, the skin of the body, the scalp (moisturizing, hair loss, sensitive scalp) and the hair (relaxing, dyeing or bleaching treatment of the roots).

Depending on the area treated or the effect desired, the device will be used to generate heating or cooling. In particular:
- heating causes an improved action of the specific active agents for the treatment of the scalp and/or hair; and
- cooling of the skin provides a soothing feeling for the scalp.

The process according to the invention may comprise, in particular, the application of a cosmetic composition to the surface of the body.

Use will be made, in particular:
- for the scalp, of a cosmetic composition chosen in particular from antidandruff products, products for preventing hair loss or for promoting regrowth of the hair, anti-seborrheic products, anti-inflammatory products, anti-irritation or soothing products, mark-preventing products or products for stimulating or protecting the scalp, and
- for the hair, of a cosmetic composition for washing the hair, for hair care or conditioning, for temporary form retention or shaping of the hair, for the temporary, semi-permanent or permanent dyeing of the hair, or for relaxing or permanent-waving.

If the device according to the invention is used to apply heat, a hair dyeing composition can in particular be applied to one or more locks of hair. In this case, the process according to the invention advantageously comprises the steps:
- of applying a cosmetic composition to keratin materials, in particular the hair, and
- of leaving the composition on for a duration of between 1 minute and 10 minutes, preferably between 2 and 7 minutes.

More particularly, the device of the invention is brought into contact with the keratin materials to be treated, in particular with the lock of hair to which the dyeing composition has been applied beforehand, for at least 2 minutes, preferably at least 4 minutes and more preferably at least 6 minutes.

The cosmetic compositions may also contain at least one cosmetic agent such as, for example, reducing agents, oxidizing agents, fatty substances, silicones, thickeners, softeners, antifoams, moisturizers, emollients, basifying agents, plasticizers, sunscreens, direct dyes or oxidation dyes, pigments, mineral fillers, clays, mineral colloids, nacres, fragrances, peptizers, preserving agents, anionic, amphoteric, zwitterionic or nonionic surfactants, fixing or nonfixing polymers, conditioning polymers, proteins and vitamins.

These agents are generally present in the composition used within the scope of the invention in an amount ranging from 0 to 20% by weight relative to the total weight of the composition.

The cosmetic compositions of the invention may be in various galenical forms, such as a lotion, a spray, a foam, a cream, a gel, a hair conditioner or a shampoo.

When the composition is used for washing and/or conditioning keratin materials, in particular the hair, it may be used in rinsed mode or non-rinsed mode.

It is also possible to use the device according to the invention to generate heating or cooling, before and/or after composition treatment, in particular for the scalp and the hair.

The invention relates in particular to a process for the pretreatment or post-treatment of keratin materials, consisting in using the device according to the invention before or after a hair dyeing or hair-root dyeing, hair bleaching or hair-root bleaching, hair permanent-waving, hair relaxing, hair regrowth, hair care or hair root care and hair conditioning treatment.

The invention also relates to a process for the pretreatment or post-treatment of the skin of the body, of the face or of the scalp, consisting in using the device according to the invention before or after a hair regrowth, care or makeup treatment.

A further subject of the invention, according to another of its aspects, independently or in combination with the foregoing, is a cosmetic kit comprising:
- a device as defined above, and
- a cosmetic composition.

Advantageously, the composition is chosen from:
- a composition for facial care or makeup,
- a composition for body care or makeup,
- a hair composition, in particular a composition for washing the hair, for hair care or conditioning, for temporary form retention or shaping of the hair, for the temporary, semi-permanent or permanent dyeing of the hair, or for relaxing or permanent-waving, in particular a composition for relaxing, dyeing or bleaching the roots and hair, and
- a composition for the scalp, in particular an antidandruff composition, a composition for preventing hair loss or for promoting regrowth of the hair, an anti-seborrheic composition, an anti-inflammatory composition, an anti-irritation or soothing composition, a mark-preventing composition or a composition for stimulating or protecting the scalp.

Advantageously, in the kit according to the invention, the cosmetic composition is a hair composition for dyeing the hair or a composition for the scalp.

The product for the skin is for example a makeup and/or care product, in particular an eye contour product, a concealer, or a product for the lips, for example a lipstick, a lip gloss or a care product for the lips.

The invention may be better understood from reading the following detailed description of nonlimiting illustrative embodiments thereof and from examining the appended drawings, in which:
- figure 1 schematically and partially shows a front view of an example of a device according to the invention,
- figure 2 is a schematic and partial side view of the device from figure 1,
- figure 3 is a schematic and partial rear view of the device from figure 1,
- figure 4 is a schematic and partial perspective exploded view of the device from figure 1,
- figure 5 is a perspective top view of the end of an application member having massaging spikes,
- figure 6 shows a photograph of an area of the scalp, comprising:
   a) a part treated just with a composition,
   b) a part treated with the same composition and the device according to the invention at ambient temperature, and
   b) a part treated with the same composition and the device according to the invention generating cooling,
- figure 7 shows the change in temperature between the center and the periphery of the three areas in figure 6.

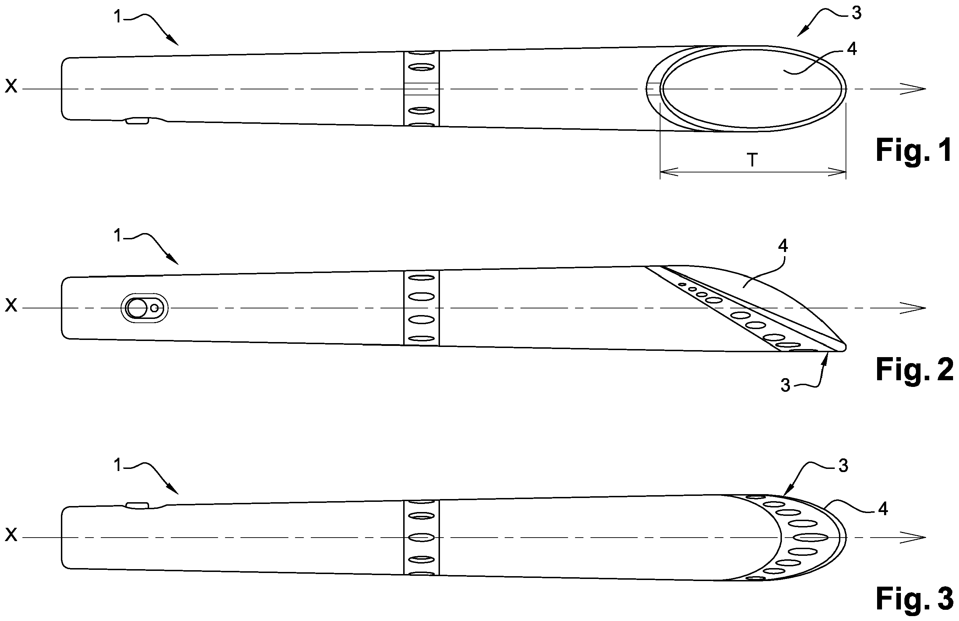

A device according to the invention is illustrated in figures 1 to 4. The device extends along a longitudinal axis X.

The device has:
- a handpiece 1 that comprises an electrical assembly 2, and
- an application member 3 that is fixed to the handpiece 1 and defines an application surface 4.

In the example shown, the heat or cold is generated by the Peltier effect.

The handpiece 1 has a cylindrical shape. It can be held between three fingers of one hand.

The electrical assembly 2 is housed entirely in the handpiece 1.

It has:
- an autonomous source or electrical power consisting of a lithium battery 50,
- a charger 51 for recharging the battery 50,
- a motor 40,
- a copper heat exchanger 30, and
- two radiators 21 and 41.

The electrical assembly 1 is actuated by a switch 52.

The Peltier effect is obtained between a copper internal mass 5 and the aluminum application member 3 for example.

The application surface 4 has an approximately planar overall shape in the example described but may have some other shape.

The application surface 4 has an area of around 6 cm² for example. Of course, if the area of the application surface is more or less extensive, being for example between 50 and 200 mm², this does not depart from the scope of the present invention.

In the example described, the application surface 4 is in the form of a convex bezel which is inclined at an angle of for example between 30 and 70° with respect to the longitudinal axis X of the device.

A largest transverse dimension T of the application member 3, for example a diameter, may be between 7 and 20 mm.

The application surface 4 has an area of around 6 cm².

The application member 3 is made of a first inorganic material, for example stainless steel. The choice of such a material makes it possible to ensure a cooling or heating effect on application.

This effect is also ensured by the fact that the application member 3 is in contact with an internal mass 5 that is made overwhelmingly of a second inorganic material. This is a heat- or cold-conducting material. In the example in question, it is copper.

According to the invention, the internal mass 5 ensures a sufficiently long cooling or heating effect at the application surface 4. This effect must be able to be felt by the user for a duration of greater than 5 minutes, or even greater than 20 minutes, and even better still greater than one hour.

The internal mass 5 is in the form of a cylinder of revolution. Its diameter may be between 1 cm and 1.5 cm. Its height may be around 1.6 cm.

The surface of the Peltier module may have a size of between 0.785 cm² and 1.7 cm².

The ratio between the application surface 4 and the surface of the Peltier module is greater than 1.

The weight of the internal mass 5 is for example around 17 grams.

In the example shown, the weight ratio between the internal mass 5 and the mass of the application member 3 may be equal to around 7.

The application member 3 is force-fitted onto the handpiece 1. It may be engaged with the handpiece 1 by any other means, for example by snap-fastening, clip-fastening or adhesive bonding.

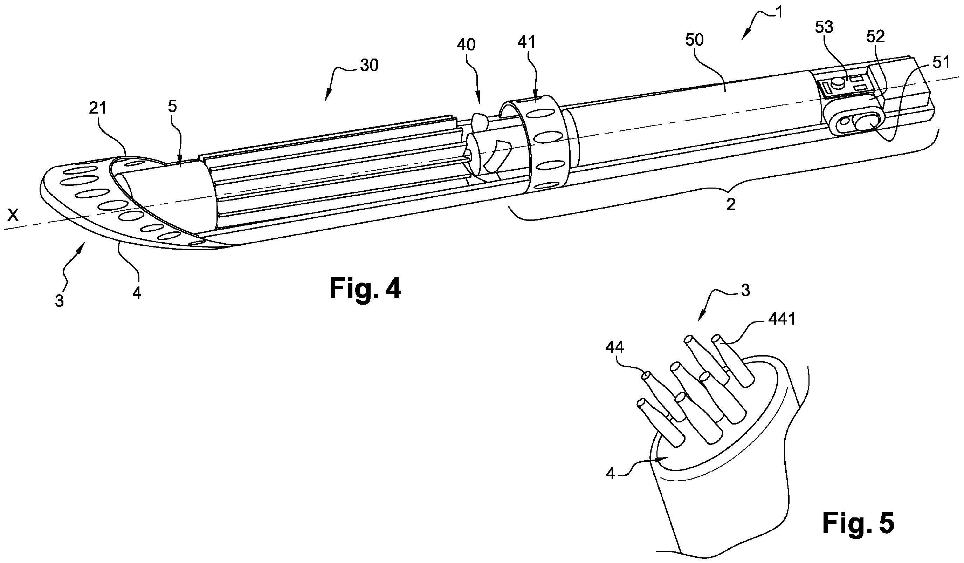

In figure 5, the application surface 4 has spikes 44. These produce a massaging effect on the keratin materials chosen. The ends 441 of the spikes come into direct contact with the skin or the scalp.

In the examples which have just been described, the application surface 4 is inclined with respect to a longitudinal axis X of the device, and this can make application easier. Moreover, the application surface 4 may be slightly convex, and beveled at its periphery.

The handpiece 1 may have a thread so as to engage with a closure cap of the device, which is not visible in figures 1 to 4.

In order to use the device of the invention, the handpiece 1 is grasped. The surface 4 is positioned in front of a chosen area of the body. The cooling of this area is started manually, for example by the user pressing the push button 53. The handpiece 1 is for example moved over the chosen keratin materials so as to progressively cool a larger surface.

The keratin materials may have been coated beforehand with a cosmetic product.

The keratin materials may be a lock of hair or the scalp for example.

In the case of a lock of hair, a dyeing product may be applied beforehand. In the case of the scalp, a hair product for promoting hair regrowth may be applied beforehand.

Of course, the invention is not limited to the examples illustrated.

It is possible for example to use other materials to produce the application member 3, for example other metals such as copper, silver, alloys and possibly a metal plated with a second metal.

It is also possible to use other materials to produce the internal mass 5.

The application surface may have a surface with a different size or shape.

The expression "*having a*" should be understood as being synonymous with *"having at least one".*

### Examples

### Example 1: Use of a device generating heating in combination with an anticellulite cream on the skin

The device from figure 1 was used to generate heating. Tests were carried out on skin explants.

Two measurements are possible:
1) Measurement of the rate and the kinetics of penetration of an active agent into the skin by microdialysis and assaying of the active agent.
2) Measurement of the quantity of active agents in the surface layers of the skin by taking strip specimens and assaying the active agent.

Tests were carried out on an area treated with a formulation in combination with the device compared with an area treated with the same formulation without a device.

The protocol used was as follows:
- Skin treated with the formulation and the device: Cellu Destock® cream from Vichy (2 mg/cm²) containing 5% by weight of caffeine is applied. Next, the skin is brought into contact with the device generating heating (40 degrees) for around 30 seconds.
- Reference: The skin is treated with the formulation without the device by applying Cellu Destock® cream from Vichy (2 mg/cm²) containing 5% by weight of caffeine.

The dialysis measurement is obtained by the prior insertion of probes into the skin and is repeated 3 times per area over an overall surface of around 20 cm².

The assays at the surface of the skin are carried out on 20 specimens of strips.
a) The tests showed the positive effects of the combination of the formulation and the device on the speed of penetration of the caffeine:
   - the caffeine penetrates twice as fast into the skin by virtue of the heat (penetration peak at 2 h with the cream and the device. In contrast, the penetration peak is at 4 hours with the cream alone),
   - the overall quantity of caffeine obtained with the heat is 1.87 times greater than the reference (cream applied without heat).
b) The tests showed the positive effects of the combination of the formulation with the device generating heating on the quantity of caffeine penetrating into the skin:
   - all of the first strips of the control group are homogeneous and indicate the presence of caffeine at the surface of the skin (10.73 µg/mL ± 0.28),
   - all of the strips combined with the device indicate the presence of caffeine but in a smaller quantity (2.88 µg/mL ± 1.44) compared with the reference.

A total of 19 strips were made, which correspond to the *stratum corneum.* These show that with the device producing heating, all of the caffeine penetrated, while in the control group caffeine was still detected (3.17 µg/mL ± 0.99).

### Example 2: Use of a device generating heating in combination with oxidation dye on the hair

The same device is used as in example 1.

In order to evaluate the activity of the heat on the dye, use was made of an oxidation dye Majirel ® from L'Oréal, shade 6.62 with a 9-volume oxidant on natural white hair with 90% white.

The device was used at a constant temperature of 56°C and the locks were placed on a plate thermostatically maintained at 33°C throughout the treatment.

Each lock was separated into two. Half of the lock was treated with the device according to different applications and the other half acted as a control.

Different applications and a control were carried out:
- Application of the device after leaving the color on for 2 minutes, passage of 2.4 or 6 minutes per side of the lock,
- Application of the device after leaving the color on for 5 minutes, passage of 2.4 or 6 minutes per side of the lock,
- Standard dyeing control, 30 minutes' leave-on time (15 minutes per side).

It was observed that the device makes it possible to activate the dye on the hair. Specifically, for the same leave-on time, the color between the halves of the lock is significantly different between the control half of the lock and the half of the lock that was treated with the device according to the invention.

### Example 3: Use of a device generating cooling in combination with a solution of Tween 21® on the inside of the arm and the scalp

The following aqueous-alcoholic solution of Tween 21® was applied to an area of the inside of the arm and an area of the scalp:

| | |
|---|---|
| Water (% RM) | 79.5% |
| Ethanol (% RM) | 20 % |
| Tween 21 (% RM) | 0.5% |

(SM stands for "*raw material").*

Each area was divided into three parts 100, 101 and 102.
- Only the composition was applied to the part 100,
- The device according to the invention was applied to the part 101 at ambient temperature for 1 minute with a slow sweeping action, in addition to the composition,
- The device according to the invention generating cooling to 5°C was applied to the part 102 for 1 minute with a slow sweeping action, in addition to the composition.

The temperature of each part 100, 101 and 102 was measured.

This temperature measurement was carried out with an infrared camera.

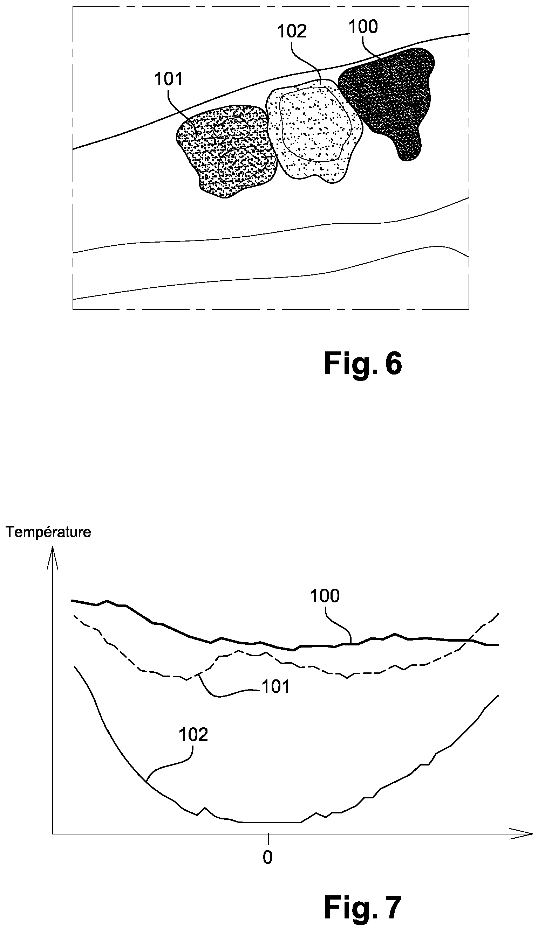

Figure 6 shows a photograph of an area of the scalp having a part 100, a part 101 and a part 102.

On each part, the color makes it possible to determine the temperature of the scalp. The lighter the color, the higher the temperature. The darker the color, the lower the temperature.

It is observed that:
- the part 100, which was treated only with the composition, is hotter than the parts 101 and 102 that were also treated with the device of the invention, and
- the part 102, which was treated with the device generating cooling, is cooler and more soothed than the part 101, which was treated with the device at ambient temperature.

It follows that the device according to the invention, generating cooling, activates cooling of the scalp in combination with a soothing active agent.

Figure 7 shows the change in temperature of each part 100, 101 and 102 from one end of each area to the other.

The following is observed:
- a very clear difference in temperature between the parts 100 and 101 on the one hand and the part 102 on the other hand,
- especially marked cooling of the scalp at the center of the part 102, and
- a difference in temperature of around 3 to 5°C between the center of the parts 100 and 101 and the center of the part 102.

It follows that the device according to the invention generating cooling, in combination with a soothing composition, has the effect of stimulating the soothing of the scalp compared with the same composition used on its own. The same effect was observed on the skin on the inside of the arm.

## Claims

1. A device for the cosmetic treatment of human keratin materials, having:
- a handpiece (1) that comprises an electrical assembly, and
- an application member (3) that is fixed to the handpiece (1) and defines an application surface (4),
the application surface (4) having an area of between 50 and 200 mm², better still between 70 and 150 mm², and
the handpiece (1) being designed to generate a thermal amplitude of greater than or equal to 5°C between the keratin materials and the application surface (4) for a period of at least 5 minutes, in particular a thermal amplitude of greater than or equal to 20°C for a period of at least 5 minutes,
the device comprises at least one Peltier module having an internal mass (5), the weight ratio between the internal mass (5) and the application member (3) is between 1 and 15, preferably 3 and 12, and more preferably between 5 and 10.

2. The device as claimed in claim 1, which has a single Peltier module.

3. The device as claimed in either of preceding claims, which has at least one heat sink (21, 41).

4. The device as claimed in any one of preceding claims, wherein the ratio between the application surface (4) and the surface of the Peltier module is strictly greater than 1, preferably between 1.02 and 2.

5. The device as claimed in any one of the preceding claims, wherein the handpiece (1) is designed to be held between three fingers of one hand.

6. The device as claimed in any one of the preceding claims, wherein the handpiece has an elongate shape, in particular a cylindrical shape.

7. The device as claimed in any one of the preceding claims, wherein the application surface is smooth.

8. The device as claimed in any one of claims 1 to 3, wherein the application surface (4) has at least one raised element (441).

9. A cosmetic process comprising the employment of a device as claimed in any one of the preceding claims, wherein:
- the device is gripped between three fingers of one hand,
- the application surface (4) is brought into contact with the keratin materials to be treated, and
- a thermal amplitude of greater than or equal to 5°C is generated between the keratin materials and the application surface (4) for a period of at least 5 minutes.

10. A cosmetic kit comprising:
- a device as claimed in any one of claims 1 to 6; and
- a cosmetic composition, in particular a hair composition for dyeing hair or promoting hair regrowth.

11. The kit as claimed in claim 10, wherein the composition is chosen from:
- a composition for facial care or makeup,
- a composition for body care or makeup,
- a hair composition, in particular a composition for washing the hair, for hair care or conditioning, for temporary form retention or shaping of the hair, for the temporary, semi-permanent or permanent dyeing of the hair, or for relaxing or permanent-waving, in particular a composition for relaxing, dyeing or bleaching the roots and hair, and
- a composition for the scalp, in particular an antidandruff composition, a composition for preventing hair loss or for promoting regrowth of the hair, an anti-seborrheic composition, an anti-inflammatory composition, an anti-irritation or soothing composition, a mark-preventing composition or a composition for stimulating or protecting the scalp.

## Patentansprüche

1. Vorrichtung zur kosmetischen Behandlung menschlicher Keratinmaterialien, die aufweist:
- ein Handstück (1), das eine elektrische Baugruppe umfasst, und
- ein Auftrageelement (3), das an dem Handstück (1) befestigt ist und eine Auftragefläche (4) definiert,
wobei die Auftragefläche (4) eine Fläche zwischen 50 und 200 mm² aufweist, besser zwischen 70 und 150 mm², und
wobei das Handstück (1) dafür gestaltet ist, eine Wärmeamplitude von größer als oder gleich 5 °C zwischen den Keratinmaterialien und der Auftragefläche (4) für einen Zeitraum von wenigstens 5 Minuten zu erzeugen, insbesondere eine Wärmeamplitude von größer als oder gleich 20 °C für einen Zeitraum von wenigstens 5 Minuten,
wobei die Vorrichtung wenigstens ein Peltier-Modul mit einer inneren Masse (5) umfasst, wobei das Gewichtsverhältnis zwischen der inneren Masse (5) und dem Auftrageelement (3) zwischen 1 und 15, vorzugsweise 3 und 12, und bevorzugter zwischen 5 und 10 beträgt.

2. Vorrichtung gemäß Anspruch 1, das ein einziges Peltier-Modul aufweist.

3. Vorrichtung gemäß einem der vorstehenden Ansprüche, das wenigstens eine Wärmesenke (21, 41) aufweist.

4. Vorrichtung gemäß einem der vorstehenden Ansprüche, wobei das Verhältnis zwischen der Auftragefläche (4) und der Oberfläche des Peltier-Moduls strikt größer als 1 ist, vorzugsweise zwischen 1,02 und 2 beträgt.

5. Vorrichtung gemäß einem der vorstehenden Ansprüche, wobei das Handstück (1) zum Halten zwischen drei Fingern einer Hand gestaltet ist.

6. Vorrichtung gemäß einem der vorstehenden Ansprüche, wobei das Handstück eine langgestreckte Form, insbesondere eine zylindrische Form, aufweist.

7. Vorrichtung gemäß einem der vorstehenden Ansprüche, wobei die Auftragefläche glatt ist.

8. Vorrichtung gemäß einem der Ansprüche 1 bis 3, wobei die Auftragefläche (4) ein erhöhtes Element (441) aufweist.

9. Kosmetisches Verfahren, umfassend den Einsatz einer Vorrichtung gemäß einem der vorstehenden Ansprüche, wobei:
- die Vorrichtung zwischen drei Fingern einer Hand gehalten wird,
- die Auftragefläche (4) mit den zu behandelnden Keratinmaterialien in Kontakt gebracht wird, und
- eine Wärmeamplitude von größer als oder gleich 5 °C zwischen den Keratinmaterialien und der Auftragefläche (4) für einen Zeitraum von wenigstens 5 Minuten erzeugt wird.

10. Kosmetisches Kit, umfassend:
- eine Vorrichtung gemäß einem der Ansprüche 1 bis 6; und
- eine kosmetische Zusammensetzung, insbesondere eine Haarzusammensetzung zum Färben von Haar oder Fördern von Haarwuchs.

11. Kit gemäß Anspruch 10, wobei die Zusammensetzung ausgewählt ist aus:
- einer Zusammensetzung zur/zum Gesichtspflege oder -makeup,
- einer Zusammensetzung zur/zum Körperpflege oder -makeup,
- einer Haarzusammensetzung, insbesondere einer Zusammensetzung zum Waschen des Haars, zur Haarpflege oder -konditionierung, zur vorübergehenden Festigung oder Gestaltung des Haars, zur vorübergehenden, halbdauerhaften oder dauerhaften Färbung des Haars oder zum Glätten oder Dauerwellen, insbesondere einer Zusammensetzung zum Glätten, Färben oder Bleichen der Wurzeln und des Haars, und
- einer Zusammensetzung für die Kopfhaut, insbesondere einer Antischuppenzusammensetzung, einer Zusammensetzung zum Verhindern von Haarverlust oder zum Fördern von Haarwuchs, einer antiseborrhoischen Zusammensetzung, einer entzündungshemmenden Zusammensetzung, einer reizhemmenden oder lindernden Zusammensetzung, einer fleckenverhindernden Zusammensetzung oder einer Zusammensetzung zum Anregen oder Schützen der Kopfhaut.

## Revendications

1. Dispositif de traitement cosmétique de matières kératiniques humaines, comportant:
- une pièce à main (1) comprenant un ensemble électrique, et
- un organe d'application (3) fixé sur la pièce à main (1) et définissant une surface d'application (4),
la surface d'application (4) ayant une superficie comprise entre 50 et 200 mm², mieux encore entre 70 et 150 mm², et
la pièce à main (1) étant agencée pour générer une amplitude thermique entre les matières kératiniques et la surface d'application (4) supérieure ou égale à 5°C pendant une durée d'au moins 5 minutes, en particulier une amplitude thermique supérieure ou égale à 20°C pendant une durée d'au moins 5 minutes, le dispositif comprenant au moins un module Peltier comportant une masse interne (5), le rapport en poids entre la masse interne (5) et l'organe d'application (3) étant compris entre 1 et 15, de préférence 3 et 12, et plus préférentiellement entre 5 et 10.

2. Dispositif selon la revendication 1, comportant un seul module Peltier.

3. Dispositif selon l'une ou l'autre des revendications précécdentes, comportant au moins un radiateur (21, 41).

4. Dispositif selon l'une quelconque des revendications précédentes, le rapport entre la surface d'application (4) et la surface du module Peltier étant strictement supérieur à 1, de préférence compris entre 1,02 et 2.

5. Dispositif selon l'une quelconque des revendications précédentes, la pièce à main (1) étant conçue pour être tenue entre trois doigts d'une main.

6. Dispositif selon l'une quelconque des revendications précédentes, la pièce à main ayant une forme allongée, en particulier une forme cylindrique.

7. Dispositif selon l'une quelconque des revendications précédentes, la surface d'application étant lisse.

8. Dispositif selon l'une quelconque des revendications 1 à 3, la surface d'application (4) comportant au moins un élément de relief (441).

9. Procédé cosmétique comprenant la mise en oeuvre d'un dispositif selon l'une quelconque des revendications précédentes, dans lequel :
- le dispositif est saisi entre trois doigts d'une main,
- la surface d'application (4) est mise en contact avec les matières kératiniques à traiter, et
- une amplitude thermique supérieure ou égale à 5°C est générée entre les matières kératiniques et la surface d'application (4) pendant une durée d'au moins 5 minutes.

10. Kit cosmétique comprenant :
- un dispositif selon l'une quelconque des revendications 1 à 6, et
- une composition cosmétique, notamment une composition capillaire pour la coloration ou la repousse des cheveux.

11. Kit selon la revendication 10, dans lequel la composition est choisie parmi :
- une composition pour le soin ou le maquillage du visage,
- une composition pour le soin ou le maquillage du corps,
- une composition capillaire notamment une composition pour le lavage des cheveux, le soin ou le conditionnement des cheveux, le maintien ou la mise en forme temporaire des cheveux, la coloration temporaire, semi-permanente ou permanente des cheveux, le défrisage ou la permanente, notamment une composition de défrisage, de coloration, de décoloration des racines et des cheveux, et
- une composition pour le cuir chevelu notamment une composition antipelliculaire, antichute ou pour la repousse des cheveux, anti séborrhée, anti-inflammatoire, anti-irritations ou apaisante, antitaches ou pour la stimulation ou la protection du cuir chevelu.
